(19) Europäisches Patentamt
European Patent Office
Office européen des brevets

(11) **EP 4 455 122 A1**

(12) **EUROPÄISCHE PATENTANMELDUNG**

(43) Veröffentlichungstag:
**30.10.2024 Patentblatt 2024/44**

(21) Anmeldenummer: 23169421.7

(22) Anmeldetag: **24.04.2023**

(51) Internationale Patentklassifikation (IPC):
**C07C 209/16** *(2006.01)* **B01J 3/00** *(2006.01)*

(52) Gemeinsame Patentklassifikation (CPC):
(C-Sets verfügbar)
**C07C 209/16; B01J 3/00** (Forts.)

(84) Benannte Vertragsstaaten:
**AL AT BE BG CH CY CZ DE DK EE ES FI FR GB
GR HR HU IE IS IT LI LT LU LV MC ME MK MT NL
NO PL PT RO RS SE SI SK SM TR**
Benannte Erstreckungsstaaten:
**BA**
Benannte Validierungsstaaten:
**KH MA MD TN**

(71) Anmelder: **BASF SE
67056 Ludwigshafen am Rhein (DE)**

(72) Erfinder:
• **SCHELLING, Heiner
67056 Ludwigshafen am Rhein (DE)**
• **PALLASCH, Hans-Juergen
67056 Ludwigshafen am Rhein (DE)**
• **BORGEL, Franz
67056 Ludwigshafen am Rhein (DE)**

(74) Vertreter: **BASF IP Association
BASF SE
GBI-C006
67056 Ludwigshafen (DE)**

(54) **VERFAHREN ZUR HERSTELLUNG VON METHYLAMINEN IN DER GASPHASE MIT VERBESSERTER REGELUNG DER REAKTOREINTRITTSTEMPERATUR**

(57) Die Erfindung betrifft ein Verfahren zur Herstellung von Methylaminen durch eine insgesamt exotherme Gasphasenreaktion aus Methanol und Ammoniak als Edukten in Gegenwart eines heterogenen Katalysators bei einem Druck im Bereich von 15 bis 35 bar. Die Edukte werden in einem oder mehreren Wärmetauschern verdampft und überhitzt bevor sie dem Reaktor zugeführt werden, wobei die Wärmetauscher wiederum mit dem wärmeren Produktgasstrom aus dem Reaktor beheizt werden. Der Produktgasstrom umfasst neben Methylaminen (Monomethylamin, Dimethylamin und Trimethylamin) auch nicht umgesetzte Edukte, Wasser und etwaige weitere Reaktionsnebenprodukte. Zur Regelung und Begrenzung der Reaktoreintrittstemperatur auf eine Temperatur im Bereich von 350°C bis 410°C wird eine geeignete Quench-Flüssigkeit in den heißen Produktgasstrom eingebracht, bevor dieser den Wärmetauschern für die Erwärmung der Edukte zugeleitet wird. Durch Verdampfung und Überhitzung der zugegebenen Quench-Flüssigkeit wird dem heißen Produktgasstrom die entsprechende Menge an Energie entzogen, wodurch dieser effizient und schnell abgekühlt wird. In der Folge ist die Temperaturdifferenz zwischen kalter und warmer Seite der Wärmetauscher kleiner, wodurch weniger Energie auf den Eduktstrom übertragen wird und so dessen Reaktoreintrittstemperatur eingestellt bzw. begrenzt werden kann.

**(Forts. nächste Seite)**

EP 4 455 122 A1

(52) Gemeinsame Patentklassifikation (CPC): (Forts.)

    C-Sets
    **C07C 209/16, C07C 211/04**

## Beschreibung

Die vorliegende Erfindung betrifft ein Verfahren zur Herstellung von Methylaminen aus Methanol und Ammoniak in einer kontinuierlichen Gasphasenreaktion an einem heterogenen Katalysator bei einem Absolutdruck im Bereich von 15 bis 35 bar. Dazu wird der Eduktstrom, umfassend die Edukte Methanol und Ammoniak, mittels Wärmetauscher verdampft und auf eine Reaktoreintrittstemperatur im Bereich von 350°C bis 410°C erwärmt. Da die Reaktion von Methanol und Ammoniak zu Methylaminen exotherm ist, wird der heiße Produktgasstrom, der neben den Methylaminen (Monomethylamin, Dimethylamin und Trimethylamin), Wasser sowie nicht umgesetzten Ammoniak und nicht umgesetztes Methanol enthält, aus dem Reaktor durch ein oder mehrere Wärmetauscher für die Erwärmung der Edukte geleitet. Das erfindungsgemäße Verfahren zeichnet sich dadurch aus, dass zur Einstellung der Reaktoreintrittstemperatur eine Quench-Flüssigkeit in den heißen Produktgasstrom eingebracht wird, bevor dieser durch die Wärmetauscher geleitet wird. Die Quench-Flüssigkeit weist bei Normaldruck einen Siedepunkt im Bereich von -40°C bis 120°C auf und verdampft beim Einbringen in den Produktgasstrom wodurch dieser effizient und gezielt auf die Temperatur abgekühlt wird, die erforderlich ist für die gewünschte Erwärmung des Eduktstroms. In einer besonderen Ausführungsform der Erfindung wird Kondensat des Produktgasstroms als Quench-Flüssigkeit für die Kühlung des Produktgasstroms eingesetzt. Die Erfindung betrifft weiter eine entsprechende Vorrichtung zur kontinuierlichen Durchführung einer insgesamt exothermen Reaktion, für die die Edukte beim Reaktoreintritt auf eine bestimmte Temperatur gebracht werden müssen Diese Vorrichtung umfassend (a) einen oder mehrere Reaktoren, denen die Edukte zugeführt und aus denen der Produktgasstrom abgezogen, (b) einen oder mehrere dem Reaktor bzw. den Reaktoren vorgeschaltete Wärmetauscher, mit denen die Edukte auf die erforderliche Reaktoreintrittstemperatur gebracht werden und durch die für den Wärmeeintrag der Produktgasstrom durchgeleitet wird, und (c) ein Messgerät für die Messung der Reaktoreintrittstemperatur der Edukte. Die erfindungsgemäße Vorrichtung zeichnet sich dadurch aus, dass sie eine geregelte Einbringung von verdampfbarer Flüssigkeit in den Produktgasstrom umfasst, bevor dieser durch einen oder mehrere der Wärmetauscher durchgeleitet wird, mittels der die Temperatur des Produktgasstroms durch Verdampfen der Flüssigkeit abgekühlt und so die gewünschte Reaktoreintrittstemperatur der Edukte eingestellt werden kann.

Methylamine sind Monomethylamin, Dimethylamin und Trimethylamin, sowie Mischungen davon.

Methylamine können in Gegenwart eines Katalysators in kontinuierlicher Gasphasenreaktion aus Methanol und Ammoniak hergestellt werden. Obschon die Reaktion insgesamt exotherm ist, müssen die Edukte für die Reaktion vorgeheizt werden. Die Vorheizung der zugeführten Edukte erfolgt üblicherweise in dem Reaktor vorgeschalteten Wärmetauschern. Zur Vorheizung kann, wie in JP 60045550 A beschrieben, der bei der Reaktion anfallende Produktgasstrom eingesetzt werden. Hierzu wird der Produktgasstrom durch die zur Vorheizung eingesetzten Wärmetauscher geleitet. Aufgrund der in Summe exothermen Reaktionen nimmt die Temperatur im Reaktor zwischen Reaktoreingang und Reaktorausgang zu. Durch die höhere Temperatur am Reaktorausgang wird bei Verwendung des Produktgasstromes zur Aufheizung der Edukte im stationären Betrieb keine weitere Energie zur Vorheizung der Edukte benötigt.

Die erforderlichen Temperaturen im Reaktor sind durch technische Begrenzungen festgelegt. Die Reaktoreintrittstemperatur muss ausreichend hoch sein, damit die Reaktion am Katalysator unmittelbar einsetzt, das Methanol weitgehend umgesetzt wird und die Reaktion dem thermischen Gleichgewicht nahekommt. Andererseits darf die Temperatur im Katalysatorbett nicht zu hoch sein, da es ansonsten zu einer vermehrten Zersetzung der Methylamine zu gasförmigen und festen Abbauprodukten kommt oder die maximal zulässige Temperatur des Reaktors überschritten wird. Eine genaue Einstellung der Reaktoreintrittstemperatur ist daher entscheidend für eine effiziente Herstellung der Methylamine.

Bei der Reaktion von Methanol und Ammoniak zu Methylaminen entsteht üblicherweise mehr Trimethylamin als benötigt wird. Überschüssiges Trimethylamin wird in der Anlage üblicherweise zurückgeführt, dem Reaktorzulauf zugegeben und im Reaktor in einer endothermen Reaktion zu Dimethylamin und Monomethylamin umgesetzt. Die Rückführung von Trimethylamin in den Reaktor führt so zu einem insgesamt weniger exothermen Verfahren. Gleiches gilt für die Rückführung der anderen Methylamine.

Bei den derzeit eingesetzten Verfahren zur Vorheizung der Edukte kann es aufgrund von Temperaturschwankungen in der Umgebung oder durch unterschiedliche Mengen an zurückgeführtem Trimethylamin, sowie bei Laständerungen zu Schwankungen bei der Reaktoreintrittstemperatur kommen. Diese Schwankungen führen in der Folge zu Schwankungen der Reaktortemperaturen. Im ungünstigsten Fall wird die Reaktortemperatur so hoch, dass sich die Methylamine zersetzen oder die maximal zulässige Reaktortemperatur überschritten wird.

Die theoretische Möglichkeit, Reaktoreintrittstemperatur des Eduktstroms dadurch zu begrenzen, dass der zur Vorheizung eingesetzte Produktgasstrom bei Erreichen der Temperaturobergrenze um die Wärmetaucher herumgeleitet wird, ist verfahrenstechnisch nicht praktikabel. Die große Menge des heißen und unter Druck stehenden Produktgasstroms schnell und kontrolliert umzuleiten würde extrem aufwendige und teure Regelventile erfordern.

WO 2005/028416 A beschreibt ein Verfahren, bei dem mittels gezielter Druckregelung der aufzuheizenden Eduktströme bzw. des abzukühlenden Reaktoraustrags die Verdampfungs- bzw. Kondensationstemperatur so eingestellt wird, dass in den Wärmetauschern genau so viel Energie übertragen wird, wie für das Erreichen der erforderlichen Reaktoreintrittstemperatur benötigt. Nachteilig an diesem Verfahren ist, dass es schwer zu regeln ist, eine träge Regelcharakteristik aufweist und zusätzlichen Druckverlust bewirkt.

Ferner erfordert das Verfahren nach WO 2005/028416 A bei erhöhter Wärmerückgewinnung durch Vorheizung der Eduktströme mit anderen geeigneten prozessintern nutzbaren Energiequellen verstärkte Regeleingriffe und damit weiter erhöhten Druckverlust. Bei Teillastbetrieb der Anlage kann sich die spezifische Wärmeübertragung erhöhen. Um diese erhöhte spezifische Wärmeübertragung auszugleichen, sind bei diesem Verfahren Regeleingriffe, die ebenfalls zusätzlichen Druckverlust erzeugen, vorzunehmen.

Der vorliegenden Erfindung lag daher die Aufgabe zugrunde, ein Verfahren zur Herstellung von Methylaminen aus Methanol und Ammoniak durch Gasphasenreaktion bereitzustellen, bei dem die Reaktoreintrittstemperatur innerhalb eines engen Bereiches schnell, effizient und gezielt und ohne Druckverlust eingestellt wird. Dabei sollte dem Verfahren möglichst keine Energie durch Wärmeabfuhr verloren gehen, und zur Vorheizung und Überhitzung der Edukte im stationären Betrieb möglichst keine Energie von außen zugeführt werden müssen.

Entsprechend betrifft die Erfindung ein Verfahren zur Herstellung von Methylaminen durch kontinuierliche Gasphasenreaktion, bei dem ein Eduktstrom, umfassend die Edukte Methanol und Ammoniak, in einem oder mehreren Wärmetauschern und optional weiteren Heizelementen verdampft und auf eine Reaktoreintrittstemperatur im Bereich von 350°C bis 410°C erwärmt wird, und anschließend einem oder mehreren Reaktoren zugeführt wird, wo er in Gegenwart eines heterogenen Katalysators bei einem Absolutdruck im Bereich von 15 bis 35 bar zu den Methylaminen Monomethylamin, Dimethylamin und Trimethylamin, umgesetzt wird, die zusammen mit Wasser und etwaigen nicht umgesetzten Edukten, sowie mit etwaigen Nebenprodukten als Produktgasstrom aus dem Reaktor abgezogen werden, dadurch gekennzeichnet, dass die Reaktoreintrittstemperatur des Eduktstroms eingestellt wird durch Betrieb von mindestens einem der Wärmetauscher mit dem Produktgasstrom, der zuvor durch Einbringung einer Quench-Flüssigkeit, die unter Normaldruck einen Siedepunkt im Bereich von -40°C bis 120°C aufweist, in den Produktgasstrom auf die dazu erforderliche Temperatur gekühlt wird.

Dem erfindungsgemäßen Verfahren zur Herstellung von Methylamin liegt also das Konzept zugrunde, die Temperatur des aus dem Reaktor kommenden Produktgasstroms vor Eintritt in den Wärmetauscher so stark abzukühlen, dass der Eduktstrom nur auf die gewünschte Reaktoreintrittstemperatur erhitzt wird, wobei die erforderliche Abkühlung des Produktgasstroms durch Einbringung und Verdampfung einer geeigneten Quench-Flüssigkeit erfolgt, die dabei vollständig verdampft. Für diesen Zweck grundsätzlich geeignet sind Flüssigkeiten, die unter den Bedingungen des Produktgasstroms (also bei Temperaturen von mindestens 350°C und einem Absolutdruck von 15 bis 35 bar) vollständig verdampfen. Dies ist der Fall bei Flüssigkeiten mit einem Siedepunkt im Bereich von -40°C bis 120°C bei Normaldruck. Dabei wird die Temperatur des Produktgasstroms durch die Verdampfung der Quench-Flüssigkeit erniedrigt, ohne dass dabei Energie abgeführt wird und verloren geht.

Zur Herstellung von Methylamin aus Methanol und Ammoniak werden die Edukte zunächst in einem oder mehreren Wärmetauschern verdampft und überhitzt. Die so auf eine Temperatur im Bereich von 350°C bis 410°C, bevorzugt im Bereich von 350°C bis 390°C und besonders bevorzugt im Bereich von 360°C bis 380°C erhitzten Edukte werden dem Reaktor mit dieser Temperatur (Reaktoreintrittstemperatur) zugeführt.

Neben Methanol und Ammoniak werden dem Reaktor üblicherweise die Methylamine zugeführt, die bei der Reaktion in einem Ausmaß entstehenden, der über dem aktuellen Bedarf liegt. Insbesondere kann es erforderlich sein, Trimethylamin, das bei der Reaktion üblicherweise in grö-ßerem Ausmaß entsteht als benötigt, wieder in die Reaktion zurückzuführen und so den Anteil an Monomethylamin und Dimethylamin an den Methylaminen für das gesamte Verfahren zu erhöhen. Durch eine solche Rückführung von Trimethylamin und die damit verbundene endotherme Transmethylierung, reduziert sich die Exothermie der Gesamtreaktion im Reaktor. Aufgrund der insgesamt exothermen Reaktion nimmt die Temperatur von Reaktoreintritt zu Reaktoraustritt zu. Damit die Temperatur im Reaktor nicht über 450°C steigt, ist die Einstellung der Reaktoreintrittstemperatur des Eduktstroms zu beachten. Hierbei gilt, dass eine eher geringe Reaktoreintrittstemperatur einzustellen ist, wenn die Reaktion mit geringer Rückführung von Methylaminen, also mit hoher Exothermie (vorzugsweise Reaktoreintrittstemperatur von 350°C bis 370°C), gefahren wird, bzw. eine eher hohe Reaktoreintrittstemperatur einzustellen ist, wenn die Reaktion mit hoher Rückführung von Methylaminen, also mit geringer Exothermie (vorzugsweise Reaktoreintrittstemperatur von 370°C bis 410°C), gefahren wird. Überschüssige Reaktionswärme kann gegebenenfalls durch Kühlung des Reaktors abgeführt werden. Vorzugsweise wird die Reaktion in dem Reaktor so gefahren, dass der Produktgasstrom bei Reaktoraustritt eine Temperatur im Bereich von 390°C bis 450°C aufweist.

Im Reaktor werden die Edukte in Gegenwart eines heterogenen Katalysators bei einem Absolutdruck im Bereich von 15 bis 35 bar zu Monomethylamin, Dimethylamin und Trimethlamin umgesetzt. Üblicherweise gilt dieser Druckbereich nicht nur für den Reaktor, sondern auch für die gesamte Zuleitung des Eduktstroms durch die Wärmetauscher und die Abführung des Produktgasstroms über die Wärmetauscher samt etwaiger Flüssigkeitsabscheider. Demnach liegt vorzugsweise auch an der Stelle der Einbringung der Quench-Flüssigkeit in den Produktgasstrom ein Absolutdruck im Bereich von 15 bis 35 bar an.

Aus dem Reaktor wird ein Produktgasstrom abgezogen, der neben den Methylaminen Monomethylamin, Dimethylamin und Trimethylamin, auch bei der Reaktion als Nebenprodukt erzeugtes Wasser enthält. Üblicherweise enthält der Produktgasstrom zudem auch nicht umgesetztes Methanol, nicht umgesetzten Ammoniak sowie weitere Nebenprodukte wie Kohlenmonoxid und Kohlendioxid.

Die Reaktion kann in einem oder in mehreren Reaktoren erfolgen. Vorzugsweise erfolgt die Reaktion in einem Reaktor. Wenn mehrere Reaktoren eingesetzt werden, dann können diese parallel oder in Reihe geschaltet sein, oder in Kombinationen davon.

Der heterogene Katalysator ist üblicherweise ein mit Siliziumdioxid dotiertes Aluminiumoxid wie beispielsweise der Katalysator N7066 von BASF. Typischerweise wird der heterogene Katalysator als Festbett eingesetzt.

Der Produktgasstrom kann anschließend in die einzelnen Reaktionsprodukte getrennt werden. Die Auftrennung erfolgt üblicherweise destillativ in mehreren Destillationskolonnen. Bei der Bildung der Methylamine entsteht meist mehr Trimethylamin als gewünscht. Der Überschuss an Trimethylamin kann dann im Eduktstrom erneut der Reaktion zugeführt werden. Die Umsetzung von Trimethylamin mit Ammoniak zu Monomethylamin und Dimethylamin oder mit Monomethylamin zu Dimethylamin ist endotherm. Entsprechende endotherme Umsetzungen finden statt, wenn die anderen Methylamine zurückgeführt werden. Für die endotherme Umsetzung wird ein Teil der bei der exothermen Reaktion von Ammoniak und Methanol zu Methylaminen freiwerdende Reaktionswärme verbraucht. Aus diesem Grund kann der Reaktor auch adiabat betrieben werden.

Bei der Wärmeabgabe zum Aufheizen des Eduktstroms wird der Produktgasstrom in den Wärmetauschern abgekühlt und zumindest teilweise kondensiert. Das Kondensat kann vorzugsweise mittels Flüssigkeitsabscheider von den gasförmigen Komponenten des abgekühlten Produktstroms abgetrennt werden. Dabei kann es sich um einen separaten, den Wärmetauschern nachgeschalteten Flüssigkeitsabscheider oder vorzugsweise um in den Wärmetauschern integrierte Flüssigkeitsabscheider handeln. Durch solche integrierten oder den einzelnen Wärmetauschern jeweils unmittelbar nachgeschalteten Flüssigkeitsabscheider (Kondensatabscheider), kann Erosion durch mitgerissene Kondensattropfen vermieden werden. Die in den Kondensatabscheidern anfallenden flüssigen Produktströme werden vorzugsweise ebenso wie der die Wärmetauscher verlassende Produktgasstrom einer Weiterbehandlung zur Abtrennung und gegebenenfalls Auftrennung der Methylamine zugeführt.

Die zum Anfahren der Reaktion erforderliche Wärme kann durch eine zusätzliche Aufheizung der Edukte mit extern beheizten Wärmetauschern bzw. elektrischen Heizelementen zugeführt werden. Sollte der Produktgasstrom den Edutkstrom im stationären Betrieb nicht ausreichend erwärmen, kann die fehlende Wärmemenge ebenfalls mit extern beheizten Wärmetauschern bzw. elektrischen Heizelementen zugeführt werden.

Als Wärmetauscher zum Vorheizen des Eduktstroms können alle dem Fachmann bekannten Wärmetauscherbauarten eingesetzt werden. Als Wärmetauscher eignen sich zum Beispiel Rohrbündelwärmetauscher, Spiralwärmetauscher oder Plattenwärmetauscher. Vorzugsweise werden Rohrbündelwärmetauscher eingesetzt. Für diesen Zweck besonders bevorzugte Rohrbündelwärmetauscher sind modifizierte Schwimmkopfwärmetauscher. Die eingesetzten Wärmetauscher können dabei im Gleichstrom, Gegenstrom oder Kreuzstrom betrieben werden. Weiterhin ist jede dem Fachmann bekannte Kombination aus Kreuz-, Gegen- und Gleichstrom möglich. So kann zum Beispiel bei Einsatz mehrerer Wärmetauscher zumindest ein Wärmetauscher im Gleichstrom betrieben werden, während die restlichen Wärmetauscher im Gegenstrom arbeiten. Die bevorzugte Betriebsart der Wärmetauscher zur Aufheizung der Edukte ist der Gegenstrom. Vorzugsweise weisen die Wärmetauscher einen integrierten Kondensatabscheider auf, für die Abtrennung von Kondensat, das bei der Abkühlung des Produktgasstroms im Wärmetauscher entsteht.

Um die Reaktoreintrittstemperatur zu regeln, werden üblicherweise an verschiedenen Stellen der Anlage zur Herstellung von Methylaminen Messwerte erfasst. So kann zum Beispiel vor der Zumischung des Methanols in den ammoniakhaltigen Eduktstrom der Durchfluss des ammoniakhaltigen Eduktstroms und der Durchfluss des Methanolstroms gemessen werden. Weiterhin kann bei Zumischung des Methanols an verschiedenen Positionen des ammoniakhaltigen Eduktstromes der Durchfluss der einzelnen Methanolteilströme gemessen werden. Bei einer Auftrennung des Produktgasstromes in mehrere Teilströme kann in jedem Teilstrom eine Durchflussmessung erfolgen. Auch kann durch eine Durchflussmessung die Menge an gegebenenfalls notwendigem zusätzlichem Dampf zur Aufheizung der Edukte gemessen werden.

Zur gezielten Regelung der Reaktoreintrittstemperatur ist es erforderlich, dass die Reaktoreintrittstemperatur des Eduktstroms gemessen wird. Neben der Reaktoreintrittstemperatur können auch die Temperatur des ammoniakhaltigen Eduktstromes, des Methanols, Temperaturen innerhalb des Reaktors und die Temperatur des Produktgasstroms am Reaktorausgang gemessen werden.

Vorzugsweise umfasst das erfindungsgemäße Verfahren einen automatischen Regelkreis, der die Einbringung von Quench-Flüssigkeit in den Produktgasstrom über ein Regelventil in Abhängigkeit von der gemessenen Reaktoreintrittstemperatur steuert. Zudem können noch weitere Messwerte, wie Durchfluss der verschiedenen Prozessströme, Reaktoraustrittstemperatur und Temperatur des Produktstroms nach Einbringung der Quench-Flüssigkeit, in den Regelkreis mit einbezogen werden, um die Steuerung der Reaktoreintrittstemperatur zu optimieren.

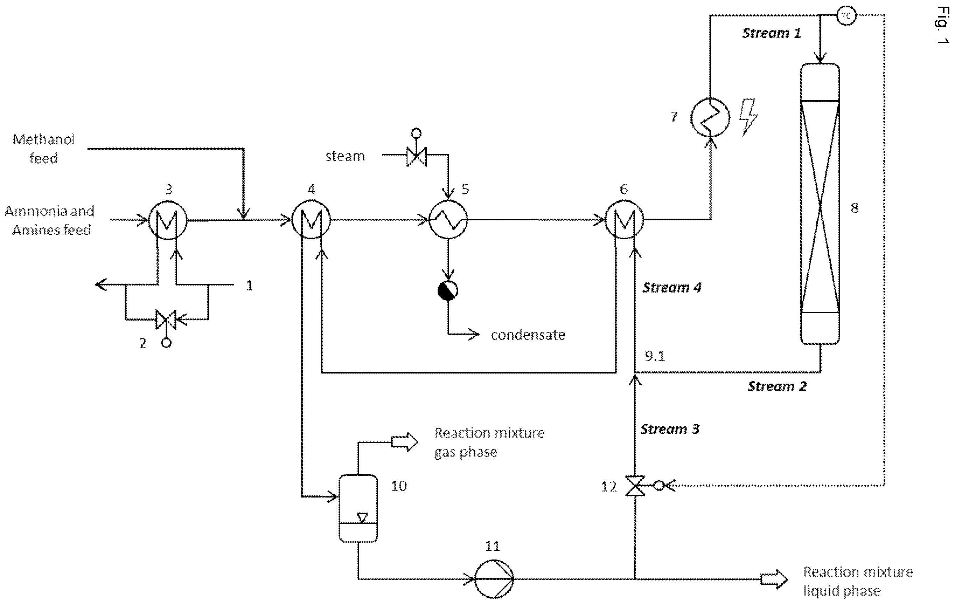

Für ein energieeffizientes Verfahren zur Herstellung von Methylaminen ist es sinnvoll im stationären Betrieb sowohl bei Teil- als auch bei Volllast den Eduktstrom ohne den Betrieb von extern beheizten Wärmetauschern oder elektrischen Heizelementen, also nur mittels Wärmetauscher die durch warme Prozessströme, insbesondere den Produktgasstrom, beheizt werden, auf Reaktoreintrittstemperatur aufzuheizen. So ist es zum Beispiel grundsätzlich möglich im ersten Wärmetauscher (3) des Verfahrens von Fig. 1 die flüssige Mischung aus Ammoniak und gegebenenfalls rückgeführten Methylaminen mit einem externen Strom zu erwärmen und teilweise zu verdampfen, vorzugsweise geschieht das aber mittels eines warmen und abzukühlenden Prozessstroms, beispielsweise einem warmen Abwasserstrom aus dem Prozess.

Aufgrund von Temperaturschwankungen in der Umgebung, durch unterschiedliche Mengen rückgeführter Methylamine oder anderen Schwankungen in der Betriebsführung, insbesondere bei Teillast, sowie Schwankungen der in die Vorwärmer (Wärmetauscher und elektrische Heizelemente für die Erwärmung, Verdampfung und Überhitzung des Eduktstroms) zugeführten Energie kann eine entsprechend energieeffiziente Auslegung des Verfahrens zu unerwünscht hohen Reaktoreintrittstemperaturen führen. Eine effiziente und schnelle Regelung der Reaktoreintrittsstemperatur ist daher für eine energieeffiziente Gestaltung des Verfahrens unerlässlich.

Bei dem erfindungsgemäßen Verfahren zur Herstellung von Methylaminen wird die Temperatur des Produktgasstroms durch die Einbringung und Verdampfung einer geeigneten Flüssigkeit (Quench-Flüssigkeit) so weit abgekühlt, dass die Temperatur der Edukte, die die ein oder mehreren mit dem Produktgasstrom beheizten Wärmetauscher verlassen, den für einen bestimmungsgemäßen Betrieb des Reaktors erforderlichen Wert nicht übersteigt. Eine geeignete Quench-Flüssigkeit ist eine Flüssigkeit, die einen Siedepunkt im Bereich von -40 bis 120°C unter Normaldruck, vorzugsweise im Bereich von -35 bis 105°C unter Normaldruck aufweist. Eine solche Quench-Flüssigkeit kann ein Reinstoff sein oder aber auch ein Gemisch mehrerer Stoffe. Bevorzugte Quench-Flüssigkeiten sind die im Verfahren bereits vorhandenen Stoffe Ammoniak, Methanol, Wasser sowie Mono-, Di- und Trimethylamin sowie Mischungen derselben. Besonders bevorzugte Quench-Flüssigkeiten sind Stoffe oder ihre Mischungen, die bei einer anschließenden Auftrennung des Reaktoraustrags zu keinem signifikanten zusätzlichen Aufwand führen wie zum Beispiel Wasser, bei der Abkühlung des Produktstroms anfallendes Kondensat oder Sumpfabzüge aus Destillationskolonnen dieser anschließenden Auftrennung. Ganz besonders bevorzugt ist als Quench-Flüssigkeit das bei der Aufheizung des Eduktstroms und der damit verbundenen Abkühlung des Produktgasstroms anfallende Kondensat.

Die Einbringung der Quench-Flüssigkeit in den Produktgasstrom erfolgt vorzugsweise durch Einspritzen mittels Pumpe und Regelventil oder durch Zufuhr mittels Regelventil und Treibstrahldüse oder durch hydrostatische Zufuhr über ein Regelventil. Besonders bevorzugt ist das Einspritzen mittels Pumpe und Regelventil. Bei der Einspritzung mittels Pumpe und Regelventil, wird durch die Pumpe der erforderliche Überdruck für die Einspritzung aufgebaut und durch das Regelventil der Einlass in den Produktgasstrom gesteuert. Bei der hydrostatischen Zufuhr sind die Elemente der Anlage so angeordnet, dass die Zufuhr der Quench-Flüssigkeit in den Produktgasstrom über den hydrostatischen Druck erfolgt. Mit dem Regelventil wird dann der Einlass in den Produktgasstrom gesteuert. Bei der Einbringung der Quench-Flüssigkeit über eine Treibstrahldüse wird die Flüssigkeit durch den Produktgasstrom selbst angesaugt und in der Düse zerstäubt. Der Einlass in den Produktgasstrom wird dabei mit einem Regelventil gesteuert. Diese Variante ist anwendbar, wenn der Produktgasstrom einen ausreichend hohen Druck aufweist.

Für die eingebrachte Quench-Flüssigkeit wird eine schnelle Verdampfung angestrebt. Dazu wird die Quench-Flüssigkeit vorzugsweise mit einer Temperatur in den Produktgasstrom eingebracht, die nur wenig unterhalb ihres Siedepunkts bei dem an der Einbringungsstelle vorliegenden Betriebsdruck liegt, besonders bevorzugt mit einer Temperatur von nicht mehr als 20°C unterhalb dieses Siedepunkts und ganz besonders bevorzugt mit einer Temperatur von nicht mehr als 10°C unterhalb dieses Siedepunkts. Zudem wird für eine schnelle Verdampfung die Quench-Flüssigkeit vorzugsweise mittels Zerstäubung in Düsen in kleine Tropfen mit spezifisch hoher Oberfläche zerteilt. Als Düsen können dafür dem Fachmann bekannte Düsenformen, wie z.B. Flachstrahldüsen, Hohlkegeldüsen, Vollkegeldüsen, Spiraldüsen, Nebeldüsen oder auch Treibstrahldüsen eingesetzt werden. Die Düsen könne dabei kombiniert sein mit einem statischen Mischer. Alternativ kann die Quench-Flüssigkeit auf großen Oberflächen, wie z.B. regellosen Schüttungen oder strukturierten Packungen, wie sie in der Destillationstechnik eingesetzt werden, verteilt werden. Die Durchströmung der regellosen Schüttungen kann im Gleich-, Kreuz- oder Gegenstrom zur Strömungsrichtung der Quench-Flüssigkeit erfolgen. In einer bevorzugten Ausführungsform des Verfahrens, bei dem die Einbringung der Quench-Flüssigkeit durch Einspritzung mittels Pumpe und Regelventil erfolgt, wird die einzubringende Quench-Flüssigkeit mittels Flüssigkeitsverteiler auf eine regellose Schüttung oder strukturierte Packung oder vorzugsweise mittels einer Düse im Produktgasstrom verteilt. In einer bevorzugten Ausführungsform des Verfahrens, bei dem die Einbringung der Quench-Flüssigkeit durch hydrostatische Zufuhr erfolgt, wird die einzubringende Quench-Flüssigkeit mittels Flüssigkeitsverteiler auf eine regellose Schüttung oder strukturierte Packung im Produktgasstrom verteilt.

Für eine Abkühlung des Produktgasstroms werden nur geringe Mengen an Quench-Flüssigkeit benötigt.

Ein Gegenstand der Erfindung ist auch eine entsprechende Vorrichtung zur kontinuierlichen Umsetzung von Edukten zu einem Produktgasstrom in einer insgesamt exothermen Reaktion, umfassend

a) einen oder mehrere Reaktoren, denen die Edukte zugeführt und aus denen der Produktgasstrom abgezogen wird,
b) einen oder mehrere den Reaktoren vorgeschaltete Wärmetauscher, mit denen die Edukte auf die erforderliche Reaktoreintrittstemperatur gebracht werden, und durch die für den Wärmeeintrag der Produktgasstrom durchgeleitet wird, und

c) ein oder mehrere Messgeräte für die Messung der Reaktoreingangstemperatur der Edukte,

dadurch gekennzeichnet, dass die Vorrichtung eine geregelte Einbringung für verdampfbare Flüssigkeiten in den Produktgasstrom umfasst, bevor dieser durch einen oder mehrere der Wärmetauscher durchgeleitet wird. Mittels dieser Einbringung kann die Temperatur des Produktgasstroms durch Verdampfen der Flüssigkeit abgekühlt, der Wärmeaustausch im Wärmetauscher begrenzt und so die gewünschte Reaktoreintrittstemperatur der Edukte eingestellt werden.

In einer bevorzugten Ausführungsform umfasst die erfindungsgemäße Vorrichtung einen oder mehrere Flüssigkeitsabscheider, mit dem von dem Produktgasstrom nach oder bei der Durchleitung durch die Wärmetauscher ein Kondensat abgetrennt wird, und eine Zuleitung, die das Kondensat der geregelten Einbringung zuführt, wo es als verdampfbare Flüssigkeit (Quench-Flüssigkeit) in den Produktgasstrom eingebracht wird.

Entsprechend ist ein Gegenstand der Erfindung eine Vorrichtung zur kontinuierlichen Umsetzung von Edukten zu einem Produktgasstrom in einer insgesamt exothermen Reaktion, umfassend

a) einen oder mehrere Reaktoren, denen die Edukte zugeführt und aus denen der Produktgasstrom abgezogen wird,
b) einen oder mehrere den Reaktoren vorgeschaltete Wärmetauscher, mit denen die Edukte auf die erforderliche Reaktoreintrittstemperatur gebracht werden, und durch die für den Wärmeeintrag der Produktgasstrom durchgeleitet wird,
c) ein oder mehrere Messgeräte für die Messung der Reaktoreingangstemperatur der Edukte, und
d) einen oder mehrere Flüssigkeitsabscheider, mit dem von dem Produktgasstrom nach oder bei der Durchleitung durch die Wärmetauscher ein Kondensat abgetrennt wird,

dadurch gekennzeichnet, dass die Vorrichtung eine geregelte Einbringung von einem Teil des Kondensats in den Produktgasstrom umfasst, bevor dieser durch einen oder mehrere der Wärmetauscher durchgeleitet wird. Mittels dieser Einbringung kann die Temperatur des Produktgasstroms durch Verdampfen des Kondensats abgekühlt, der Wärmeaustausch im Wärmetauscher begrenzt und so die gewünschte Reaktoreintrittstemperatur der Edukte eingestellt werden.

In einer bevorzugten Ausführungsform der erfindungsgemäßen Vorrichtung umfasst die geregelte Einbringung der verdampfbaren Flüssigkeit, bzw. des Kondensats in den Produktgasstrom (a) eine Pumpe mit Regelventil oder (b) ein Regelventil mit Treibstrahldüse oder (c) eine hydrostatische Zuleitung über ein Regelventil. Um eine schnellere Verdampfung der verdampfbaren Flüssigkeit, bzw. des Kondensats und damit eine schnellere Abkühlung des Produktgasstroms zu gewährleisten, kann die erfindungsgemäße Vorrichtung eine Düse für die Einbringung der verdampfbaren Flüssigkeit, bzw. des Kondensats in den Produktgasstrom umfassen. Mittels der Düse kann die verdampfbare Flüssigkeit, bzw. das Kondensat in Form von kleinen Tropfen, die aufgrund ihrer relativ großen Oberfläche schnell verdampfen, in den Produktgasstrom eingebracht werden. Eine Düse eignet sich insbesondere für eine Einbringung von Kondensat per Pumpe und Regelventil. Zudem ist eine Düse inhärentes Element einer Einbringung per Regelventil und Treibstrahldüse. Alternativ kann die erfindungsgemäße Vorrichtung an der Stelle, an der die verdampfbare Flüssigkeit, bzw. das Kondensat in den Produktgasstrom eingebracht wird, auch eine regellose Schüttung oder eine strukturierte Packung umfassen, auf die die eingebrachte verdampfbare Flüssigkeit, bzw. das eingebrachte Kondensat mittels Flüssigkeitsverteiler verteilt wird. Durch das Verteilen der verdampfbaren Flüssigkeit, bzw. des Kondensats auf die große Oberfläche einer solchen Schüttung oder Packung wird ebenfalls eine schnelle Verdampfung ermöglicht. Eine solche Schüttung oder Packung eignet sich insbesondere für eine Einbringung von verdampfbarer Flüssigkeit, bzw. Kondensat per hydrostatischer Zuleitung über ein Regelventil, aber auch für eine Einbringung von verdampfbarer Flüssigkeit, bzw. Kondensat per Pumpe und Regelventil. Entsprechend ist diese bevorzugte Ausführungsform dadurch gekennzeichnet,

- dass bei einer Einbringung der verdampfbaren Flüssigkeit, bzw. des Kondensats per Pumpe mit Regelventil die verdampfbare Flüssigkeit, bzw. das Kondensat über einen Flüssigkeitsverteiler und eine regellose Schüttung oder strukturierte Packung oder vorzugsweise über eine Düse im Produktgasstrom verteilt wird, beziehungsweise

- dass bei einer Einbringung der verdampfbaren Flüssigkeit, bzw. des Kondensats per hydrostatischer Zuleitung über ein Regelventil die verdampfbare Flüssigkeit, bzw. das Kondensat über einen Flüssigkeitsverteiler und eine regellose Schüttung oder eine strukturierte Packung im Produktgasstrom verteilt wird.

In einer besonderen Ausführungsform der erfindungsgemäßen Vorrichtung, durchläuft das Kondensat vor der Rückführung in den Produktgasstrom ein oder mehrere Aufreinigungsgeräte. Diese Aufreinigungsgeräte sind vorzugsweise Destillationskolonnen, in denen Methylamine über Kopf abgezogen werden, und deren Sumpfprodukt dann als Kondensat in den Produktgasstrom zurückgeführt wird. Alternativ oder ergänzend dazu kann auch Kondensat in den Produktgasstrom eingebracht werden, das sich in solchen Aufreinigungsgeräten aus einem gasförmig zugeleiteten Produktgasstrom bildet, also beispielsweise das flüssige Sumpfprodukt einer Destillationskolonne, in die der gasförmige Produktgasstrom zugeleitet wird und aus denen Methylamine über Kopf abgezogen werden.

Die Erfindung wird durch die folgenden Abbildungen veranschaulicht:

Die Abbildungen Fig. 1 bis Fig. 3 zeigen die Fließbilder bestimmter Ausführungsformen des erfindungsgemäßen Verfahrens zur Herstellung von Methylaminen aus Methanol und Ammoniak.

Figur 1:

Bei dem in Fig. 1 dargestellten Verfahren zur Herstellung von Methylaminen bildet ein erster Eduktstrom ("*Ammonia and Amines feed*") den Zulauf zu einem ersten Vorwärmer (3). Der Zulauf kann neben Ammoniak auch rückgeführte Nebenprodukte sowie überschüssige Methylamine aus dem Verfahren enthalten. Dieser Eduktstrom wird im ersten Vorwärmer (3) erwärmt. Die zugeführte Wärme wird von einem warmen abzukühlenden Prozessstrom (1) bereitgestellt. Um die übertragene Wärme gegebenenfalls zu begrenzen, kann der warme Prozessstrom ganz oder teilweise über den Umgang mit dem Regelventil (2) um den Vorwärmer (3) herumgeleitet werden. Dieser vorgewärmte erste Zulauf wird anschließend mit dem Methanolzulauf ("*Methanol feed*") vermischt und dem zweiten Vorwärmer (4) zugeführt. Im Weiteren wird dieser Strom als Eduktstrom bezeichnet. Der zweite Vorwärmer (4) wird mit dem Produktgasstrom ("*Stream* 4") beheizt. Der so weiter erwärmte Eduktstrom wird durch einen dritten Vorwärmer (5), der mit Dampf ("*steam*") beheizt wird, geführt. Dieser dient vor allem der Aufheizung des Eduktstroms während des Anfahrprozesses. Er wird außerdem eingesetzt, wenn in den beiden vorangehenden Vorwärmer (3 und 4) nicht ausreichend Wärme übertragen wird. Im vierten Vorwärmer (6) wird der Eduktstrom auf die Reaktoreintrittstemperatur überhitzt. Dieser Vorwärmer (6) wird mit dem aus dem Reaktor kommenden Produktgasstrom ("*Stream 4*") beheizt. Der auf Reaktoreintrittstemperatur erhitzte Eduktstrom durchströmt schließlich einen letzten elektrisch betriebenen Vorwärmer (7) bevor er dem Reaktor (8) zugeführt wird ("*Stream 1*"). Dieser letzte Vorwärmer (7) dient vor allem der Aufheizung des Eduktstroms während des Anfahrprozesses. Er wird außerdem eingesetzt, wenn der Eduktstrom in den anderen Vorwärmern nicht die notwendige Reaktoreingangstemperatur erreicht. Während es sich also bei den ersten Vorwärmern (3, 4, 5 & 6) um mit externen (5 & ggf. 3) oder internen (4, 6 & ggf. 3) Strömen beheizte Wärmetauscher handelt, handelt es sich bei dem letzten Vorwärmer (7) und ein elektrisches Heizelement. Im Reaktor (8) erfolgt neben der exothermen Umsetzung von Methanol und Ammoniak zu Monomethylamin, Dimethylamin und Trimethylamin auch die Umsetzung von nicht benötigtem, rückgeführtem Trimethylamin zu Dimethylamin und Monomethylamin in einer endothermen Reaktion. Insgesamt sind die im Reaktor (8) ablaufenden Reaktionen jedoch exotherm. Aus diesem Grund nimmt die Temperatur über den Reaktor (8) zu. Die Temperatur des Reaktoraustrags ("*Stream 2*") ist höher als die am Reaktorzulauf ("*Stream 1*") erforderliche. Daher kann der Produktgasstrom des Reaktoraustrags in den Vorwärmern (6 & 4) zur Erwärmung des Reaktorzulaufs eingesetzt werden. Dazu wird der Produktgasstrom ("*Stream 4*") im Gegenstrom durch den vierten Vorwärmer (6) und den zweiten Vorwärmer (4) geführt. Nach dem zweiten Vorwärmer (4) wird der abgekühlte und inzwischen zweiphasige Produktstrom in einem Flüssigkeitsabscheider (10) in eine flüssige und eine gasförmige Phase getrennt. Der den Flüssigkeitsabscheider (10) verlassende Gasstrom wird der Aufarbeitung zugeführt. Ein Teil des Kondensats ("*Stream 3*") aus dem Flüssigkeitsabscheider (10) wird über eine Pumpe (11) und ein Regelventil (12) an einer dem vierten Vorwärmer (6) vorgelagerten Stelle (9.1) in den Reaktoraustrag (*"Stream 2"*) eingespritzt. Die eingespritzte Flüssigkeit verdampft und entzieht dem Reaktoraustag die dafür benötigte Verdampfungswärme, wodurch sich die Temperatur des zum vierten Vorwärmer (6) führenden Produktgasstroms ("*Stream 4*") erniedrigt. Aufgrund der so verringerten Temperatur wird im vierten Vorwärmer (6) eine geringere Wärmemenge auf den zu erhitzenden Reaktorzulauf übertragen. Zusammen mit einer Temperaturmessung im Zulauf zum Reaktor (8) kann so eine Temperaturregelung aufgebaut werden. Dadurch wird eine schnelle, unmittelbare Regelung der Reaktoreintrittstemperatur erreicht. Das nicht für die Temperaturregelung benötigte Kondensat aus dem Flüssigkeitsabscheider (10) wird ebenfalls der Aufarbeitung der Methylamine zugeführt.

Neben der hier dargestellten Ausführungsvariante mit insgesamt fünf Vorwärmern (3, 4, 5, 6 und 7) ist eine Vielzahl von Variationen bezüglich Anzahl, Anordnung und Verschaltung von Wärmetauschern zur Vorheizung des Eduktstroms möglich.

Statt des hier dargestellten Betriebs der Vorwärmer im Gegenstrom ist auch ein Betrieb im Gleichstrom oder Kreuzstrom möglich, sowie jegliche Kombination aus Gegenstrom, Gleichstrom oder Kreuzstrom.

Figur 2:

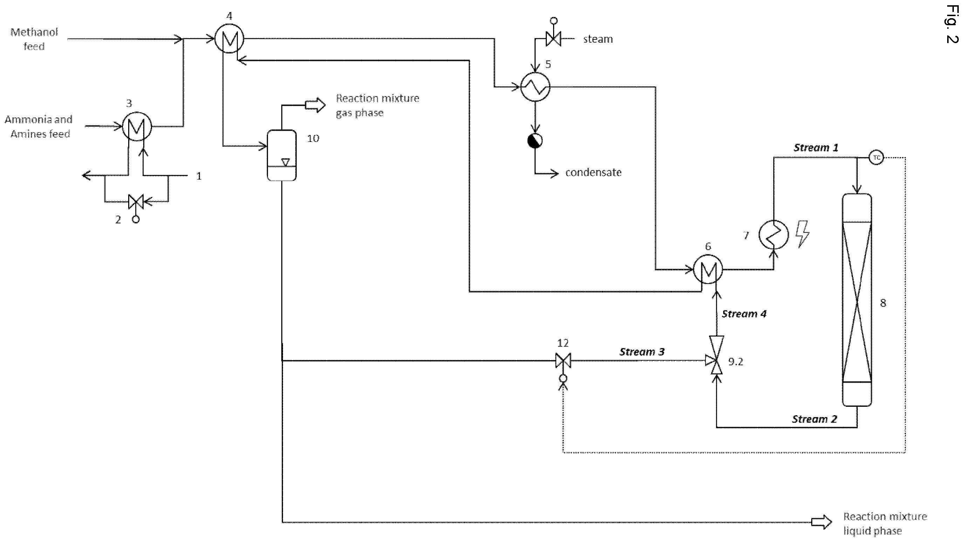

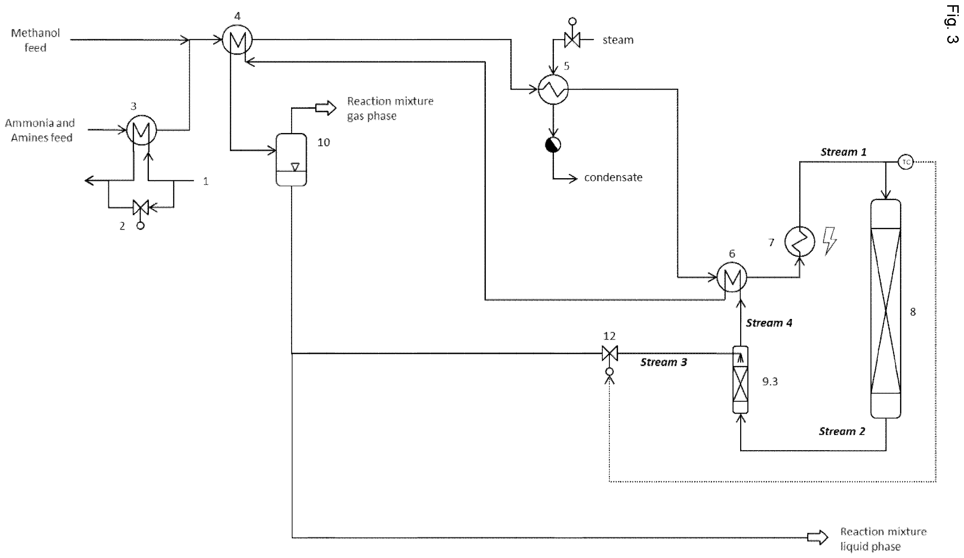

Bei dem in Fig. 2 dargestellten Verfahren handelt es sich um eine Variante des in Fig. 1 abgebildeten Verfahrens zur Herstellung von Methylaminen, bei der die Flüssigkeit aus dem Flüssigkeitsabscheider (10) über eine Treibstrahldüse (9.2) angesaugt und in der Düse zerstäubt wird. Als Treibmittel wird dabei der Produktgasstrom (*"Stream 2"*) selbst verwendet. Diese Variante kann angewandt werden, wenn der Produktgasstrom einen ausreichend hohen Druck aufweist. Figur 3: Bei dem in Fig. 3 dargestellten Verfahren handelt es sich um eine Variante des in Figur 1 abgebildeten Verfahrens zur Herstellung von Methylaminen, bei der die Vorwärmer in der Anlage so angeordnet sind, dass die Flüssigkeit aus dem Flüssigkeitsabscheider (10) hydrostatisch in den Produktgasstrom (*"Stream 2"*) eingebracht werden kann. Um eine möglichst große Flüssigkeitsoberfläche zu erzeugen, wird die Flüssigkeit mittels Flüssigkeitsverteiler auf einer regellosen Schüttung oder strukturierten Packung (9.3) verteilt, wie es entsprechend auch aus der Destillationstechnik bekannt ist.

Beispiele:

Das folgende Beispiel veranschaulicht den Effekt der erfindungsgemäßen Einbringung von Quench-Flüssigkeit in den Produktgasstrom eines Verfahrens zu Herstellung von Methylaminen wie es in Fig. 1 bis Fig. 3 näher beschrieben ist. Der Reaktoraustrag (Produktgasstrom) weist typischerweise eine Zusammensetzung auf, wie in Tab. 1 zusammengestellt. Diese Zusammensetzung kann je nach Ausführung und Betriebsweise des Verfahrens variieren.

Tab. 1:

| Typische Zusammensetzung des Produktgasstroms für das erfindungsgemäße Verfahrens zur Herstellung von Methylaminen | | |
|---|---|---|
| Wasserstoff | < 1,0 | Gew.-% |
| Stickstoff | < 1,0 | Gew.-% |
| Ammoniak | 25 - 37 | Gew.-% |
| Dimethylether | < 1,0 | Gew.-% |
| Monomethylamin | 9 - 13 | Gew.-% |
| Dimethylamin | 11 - 16 | Gew.-% |
| Trimethylamin | 23 - 35 | Gew.-% |
| Methanol | < 2,5 | Gew.-% |
| Wasser | 14 - 18 | Gew.-% |

Typischerweise wird eine Reaktoreintrittstemperatur von beispielsweise 360°C eingestellt. Bei einer adiabaten Temperaturerhöhung im Reaktor von ca. 50°C bis 80°C, wie sie für das Verfahren üblich ist, ergibt sich eine typische Reaktoraustrittstemperatur von 410°C bis 440°C. Somit führt bereits eine Temperaurerniedrigung des Reaktoraustrags (Produktgasstrom) von beispielsweise 10°C zu einer signifikanten Verringerung der Temperaturdifferenz zwischen Zulauf- und Austragsstrom und damit zu einer ebenfalls beträchtlichen Verringerung der in der Vorheizerstrecke übertragenen Energie. Erfindungsgemäß wird eine solche Temperaturerniedrigung durch Einbringen einer geeigneten Quench-Flüssigkeit in den Produktgasstrom erreicht. Anhand der Prozessbedingungen (Reaktoraustrag: 20,8 bar, 427,2°C, Temperatur der Quench-Flüssigkeit: s. Tabelle 2) und der thermodynamischen Daten von Produktgasstrom und eingebrachter Quench-Flüssigkeit, lässt sich die Menge an Quench-Flüssigkeit bestimmen, die erforderlich ist, eine bestimmte Menge an Produktgasstrom entsprechend abzukühlen. Diese Ergebnisse sind in Tab. 2 für verschiedene infrage kommende Quench-Flüssigkeiten zusammengestellt.

Tab. 2:

| Bestimmung der in den Reaktoraustrag einzubringenden Flüssigkeitsmenge für verschiedene infrage kommende Quench-Flüssigkeiten, die erforderlich ist, um die Temperatur des Reaktoraustrags um 10°C zu verringern. | | |
|---|---|---|
| Quench-Flüssigkeit | Temperatur Quench-Flüssigkeit | Menge Quench-Flüssigkeit |
| | °C | $\dfrac{kg}{(t\ Reaktoraustrag * 10°C)}$ |
| Ammoniak | 40 - 50 | 14,6 - 15,8 |

(fortgesetzt)

| Bestimmung der in den Reaktoraustrag einzubringenden Flüssigkeitsmenge für verschiedene infrage kommende Quench-Flüssigkeiten, die erforderlich ist, um die Temperatur des Reaktoraustrags um 10°C zu verringern. | | |
|---|---|---|
| Quench-Flüssigkeit | Temperatur Quench-Flüssigkeit | Menge Quench-Flüssigkeit |
| Monomethylamin | 75 - 85 | 18,1 - 19,5 |
| Dimethylamin | 100 - 110 | 22,2 - 24,0 |
| Trimethylamin | 105 - 115 | 25,1 - 27,0 |
| Methanol | 155 - 165 | 18,9 - 20,5 |
| Wasser | 200 - 210 | 10,9 - 11,8 |
| Kondensat | 83 - 93 | 14,2 - 15,9 |

Kondensat bedeutet hier das Kondensat, welches anfällt, wenn der Reaktoraustrag in den Wärmetauschern der Vorheizerstrecke durch Wärmeübertragung an den Reaktorzulauf abgekühlt wird. In dem Verfahren wie es in Fig. 1 bis 3 beschrieben ist, wird Kondensat zur Reduzierung der Temperatur des Produktgasstroms eingesetzt. Grundsätzlich kommen aber auch die anderen in Tab. 2 aufgeführten Quench-Flüssigkeiten zur Reduzierung der Temperatur infrage. Für einen ausgewählten Betriebszustand des Verfahrens gemäß Fig. 1 sind in Tab. 3 als Beispiel die Daten (Zusammensetzungen, Druck, Temperatur und Aggregatzustand) für eine Abkühlung des Reaktoraustrags von 427,2°C auf 370°C mit Kondensat zusammengestellt.

Tab. 3:

| Zusammensetzungen und physikalische Parameter der Ströme (*Stream* 1 bis 4) für eine Durchführung des Verfahrens zur Herstellung von Methylaminen, wie in Fig. 1 dargestellt, bei dem der Produktgasstrom durch Einbringen von Kondensat so weit abgekühlt wird, dass die erforderliche Reaktoreintrittstemperatur des Eduktsstroms gewahrt bleibt. | | | | | |
|---|---|---|---|---|---|
| | | *Stream 1* | *Stream 2* | *Stream 3* | *Stream 4* |
| | Molmasse (g) | Gew.-% | Gew.-% | Gew.-% | wt% |
| Wasserstoff | 2 | 0,00 | 0,01 | 0,00 | 0,01 |
| Stickstoff | 28 | 0,01 | 0,05 | 0,00 | 0,05 |
| Ammoniak | 17 | 38,92 | 30,18 | 24,36 | 29,71 |
| Dimethylether | 46 | 0,09 | 0,09 | 0,05 | 0,09 |
| Monomethylamin | 31 | 4,34 | 11,05 | 11,65 | 11,10 |
| Dimethylamin | 45 | 0,96 | 13,25 | 13,49 | 13,27 |
| Trimethylamin | 59 | 27,65 | 28,93 | 22,41 | 28,41 |
| Methanol | 32 | 27,00 | 0,54 | 0,92 | 0,57 |
| Wasser | 18 | 1,03 | 15,91 | 27,12 | 16,80 |
| Σ | kg/h | 1000,00 | 1000,00 | 85,92 | 1085,92 |
| Druck (abs) | bar | 21,8 | 20,8 | 27,5 | 20,8 |
| Temperatur | °C | 360,0 | 427,2 | 88,0 | 370,0 |
| Aggregatzustand | | gasförmig | gasförmig | flüssig | gasförmig |

Mit der in diesem Beispiel gezeigten Temperaturerniedrigung wird die Temperaturdifferenz zwischen dem den letzten Wärmetauscher verlassenden und dem in den Wärmetauscher eintretenden Prozessstrom von 67,2°C auf 10°C verringert.

Die für die Abkühlung um 57,2°C benötige Menge von 85,92 kg Kondensat pro Tonne Reaktoraustrag entspricht ca. 15 kg Kondensat pro Tonne Reaktoraustrag pro 10°C Abkühlung.

**Patentansprüche**

1. Ein Verfahren zur Herstellung von Methylaminen durch kontinuierliche Gasphasenreaktion, bei dem ein Eduktstrom, umfassend die Edukte Methanol und Ammoniak, in einem oder mehreren Wärmetauschern und optional weiteren Heizelementen verdampft und auf eine Reaktoreintrittstemperatur im Bereich von 350°C bis 410°C erwärmt wird, und anschließend einem oder mehreren Reaktoren zugeführt wird, wo er in Gegenwart eines heterogenen Katalysators bei einem Absolutdruck im Bereich von 15 bis 35 bar zu den Methylaminen Monomethylamin, Dimethylamin und Trimethylamin, umgesetzt wird, die zusammen mit Wasser und etwaigen nicht umgesetzten Edukten, sowie mit etwaigen Nebenprodukten als Produktgasstrom aus dem Reaktor abgezogen werden, **dadurch gekennzeichnet, dass** die Reaktoreintrittstemperatur des Eduktstroms eingestellt wird durch Betrieb von mindestens einem der Wärmetauscher mit dem Produktgasstrom, der zuvor durch Einbringung einer Quench-Flüssigkeit, die unter Normaldruck einen Siedepunkt im Bereich von -40°C bis 120°C aufweist, in den Produktgasstrom auf die dazu erforderliche Temperatur gekühlt wird.

2. Das Verfahren gemäß Anspruch 1, **dadurch gekennzeichnet, dass** die Reaktoreintrittstemperatur kontinuierlich gemessen wird.

3. Das Verfahren gemäß Anspruch 1 oder 2, **dadurch gekennzeichnet, dass** die Reaktion in dem Reaktor so gefahren wird, dass der Produktgasstrom bei Reaktoraustritt eine Temperatur im Bereich von 390°C bis 450°C aufweist.

4. Das Verfahren gemäß einem der Ansprüche 1 bis 3, **dadurch gekennzeichnet, dass** die Verdampfung und Einstellung der Temperatur der Edukte mittels zwei oder mehr Wärmetauscher erfolgt und die Einbringung der Quench-Flüssigkeit in den Produktgasstrom vor dem für den Produktgasstrom ersten Wärmetauscher erfolgt.

5. Das Verfahren gemäß einem der Ansprüche 1 bis 4, **dadurch gekennzeichnet, dass** die Quench-Flüssigkeit eine oder mehrere Komponenten umfasst, die ausgewählt sind aus der Gruppe bestehend aus Monomethylamin, Dimethylamin, Trimethylamin, Ammoniak, Methanol, Wasser.

6. Das Verfahren gemäß einem der Ansprüche 1 bis 5, **dadurch gekennzeichnet, dass** der Produktgasstrom ganz oder teilweise kondensiert wird und anschließend die entstandenen Methylamine über ein oder mehrere Destillationskolonnen gereinigt und gegebenenfalls in Fraktionen einzelner Methylamine oder Methylamin-Zusammensetzungen aufgetrennt werden.

7. Das Verfahren gemäß Anspruch 6, **dadurch gekennzeichnet, dass** eine Trimethylamin enthaltende oder Trimethylamin-angereicherte Fraktion der Reaktion in dem Reaktor zurückgeführt wird.

8. Das Verfahren gemäß einem der Ansprüche 1 bis 7, **dadurch gekennzeichnet, dass** als Quench-Flüssigkeit ein im Verfahren vorkommender Flüssigkeitsstrom verwendet wird.

9. Das Verfahren gemäß einem der Ansprüche 1 oder 7, **dadurch gekennzeichnet, dass** der Produktgasstrom abgekühlt wird und dabei ganz oder teilweise kondensiert, und als Quench-Flüssigkeit das anfallende Kondensat eingesetzt wird.

10. Das Verfahren gemäß Anspruch 6 oder 7, **dadurch gekennzeichnet, dass** als Quench-Flüssigkeit ein Sumpfabzug aus einer oder mehrerer der Destillationskolonnen eingesetzt wird.

11. Das Verfahren gemäß einem der Ansprüche 1 bis 10, **dadurch gekennzeichnet, dass** das Einbringen der Quench-Flüssigkeit in den Produktgasstrom durch

    a) Einspritzen mittels Pumpe und Regelventil,
    b) Zufuhr mittels Regelventils und Treibstrahldüse, oder
    c) hydrostatische Zufuhr über ein Regelventil

    erfolgt.

12. Das Verfahren gemäß Anspruch 11, **dadurch gekennzeichnet, dass** die eingespritzte Quench-Flüssigkeit mittels Flüssigkeitsverteiler auf eine regellose Schüttung oder strukturierte Packung oder vorzugsweise mittels einer Düse im Produktgasstrom verteilt wird, beziehungsweise dass die hydrostatisch zugeführte Quench-Flüssigkeit mittels Flüssigkeitsverteiler auf eine regellose Schüttung oder strukturierte Packung im Produktgasstrom verteilt wird.

13. Eine Vorrichtung zur kontinuierlichen Umsetzung von Edukten zu einem Produktgasstrom in einer insgesamt exothermen Reaktion, umfassend

a) einen oder mehrere Reaktoren, denen die Edukte zugeführt und aus denen der Produktgasstrom abgezogen wird,
b) einen oder mehrere den Reaktoren vorgeschaltete Wärmetauscher, mit denen die Edukte auf die erforderliche Reaktoreintrittstemperatur gebracht werden, und durch die für den Wärmeeintrag der Produktgasstrom durchgeleitet wird, und
c) ein oder mehrere Messgeräte für die Messung der Reaktoreintrittstemperatur der Edukte,

**dadurch gekennzeichnet, dass** die Vorrichtung eine geregelte Einbringung für verdampfbare Flüssigkeiten in den Produktgasstrom umfasst, bevor dieser durch einen oder mehrere der Wärmetauscher durchgeleitet wird, mittels der die Temperatur des Produktgasstroms durch Verdampfen der Flüssigkeit abgekühlt, der Wärmeaustausch im Wärmetauscher begrenzt und so die gewünschte Reaktoreintrittstemperatur der Edukte eingestellt werden kann.

14. Die Vorrichtung nach Anspruch 13, **dadurch gekennzeichnet, dass** die erfindungsgemäße Vorrichtung einen oder mehrere Flüssigkeitsabscheider, mit dem von dem Produktgasstrom nach oder bei der Durchleitung durch die Wärmetauscher ein Kondensat abgetrennt wird, und eine Zuleitung, die das Kondensat der geregelten Einbringung zuführt, wo es als verdampfbare Flüssigkeit in den Produktgasstrom eingebracht wird, umfasst.

15. Die Vorrichtung nach Anspruch 13 oder 14, **dadurch gekennzeichnet, dass** die Vorrichtung für die Einbringung der verdampfbaren Flüssigkeit in den Produktgasstrom

a) eine Pumpe mit Regelventil,
b) ein Regelventil mit Treibstrahldüse, oder
c) eine hydrostatische Zuleitung über ein Regelventil

umfasst.

16. Die Vorrichtung nach Anspruch 15, **dadurch gekennzeichnet,**

- **dass** bei einer Einbringung der verdampfbaren Flüssigkeit per Pumpe mit Regelventil die verdampfbare Flüssigkeit über einen Flüssigkeitsverteiler und eine regellose Schüttung oder strukturierte Packung oder vorzugsweise über eine Düse im Produktgasstrom verteilt wird, beziehungsweise
- **dass** bei einer Einbringung der verdampfbaren Flüssigkeit per hydrostatischer Zuleitung über ein Regelventil die verdampfbare Flüssigkeit über einen Flüssigkeitsverteilter und eine regellose Schüttung oder eine strukturierte Packung im Produktgasstrom verteilt wird.

Fig. 1

Fig. 2

Fig. 3

Europäisches
Patentamt

European
Patent Office

Office européen
des brevets

# EUROPÄISCHER RECHERCHENBERICHT

**Nummer der Anmeldung**

**EP 23 16 9421**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2005/028416 A2 (BASF AG [DE]; REUTEMANN WERNER [DE] ET AL.) 31. März 2005 (2005-03-31) * Ansprüche 1,4 * * Seite 3, Absatz 3 – Absatz 5 * * Seite 4, Absatz 2 * * Seite 7, Absatz 4 * * Seite 5, Absatz 2 * * Seite 2, Absatz 1 * ————— | 1,2,4-8 | INV. C07C209/16 B01J3/00 |
| A,D | JP S60 45550 A (NITTO CHEMICAL INDUSTRY CO LTD) 12. März 1985 (1985-03-12) * Zusammenfassung * ————— | 1-16 | |
| A,D | CN 115 193 344 A (ANYANG JIUTIAN FINE CHEMICAL CO LTD) 18. Oktober 2022 (2022-10-18) * Zusammenfassung * ————— | 1-16 | |
| | | | **RECHERCHIERTE SACHGEBIETE (IPC)** C07C B01J |

Der vorliegende Recherchenbericht wurde für alle Patentansprüche erstellt

| Recherchenort | Abschlußdatum der Recherche | Prüfer |
|---|---|---|
| Den Haag | 13. Oktober 2023 | Seitner, Irmgard |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

......................................................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 23 16 9421

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-10-2023

| Im Recherchenbericht angeführtes Patentdokument | Datum der Veröffentlichung | Mitglied(er) der Patentfamilie | | Datum der Veröffentlichung |
|---|---|---|---|---|
| WO 2005028416 A2 | 31-03-2005 | AT | 541825 T | 15-02-2012 |
| | | BR | PI0413792 A | 07-11-2006 |
| | | CN | 1856462 A | 01-11-2006 |
| | | DE | 10344283 A1 | 21-04-2005 |
| | | EP | 1670746 A2 | 21-06-2006 |
| | | JP | 4478683 B2 | 09-06-2010 |
| | | JP | 2007506700 A | 22-03-2007 |
| | | KR | 20060097002 A | 13-09-2006 |
| | | MX | PA06002571 A | 05-06-2006 |
| | | US | 2007270614 A1 | 22-11-2007 |
| | | WO | 2005028416 A2 | 31-03-2005 |
| JP S6045550 A | 12-03-1985 | KEINE | | |
| CN 115193344 A | 18-10-2022 | KEINE | | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- JP 60045550 A

- WO 2005028416 A